# EUROPÄISCHE PATENTSCHRIFT

(11) **EP 1 466 566 B1**
(45) Veröffentlichungstag und Bekanntmachung des Hinweises auf die Patenterteilung: **05.12.2007**
(21) Anmeldenummer: 04008386.7
(22) Anmeldetag: 07.04.2004
(51) Int. Cl.: A61B 17/60, A61M 1/28, A61F 2/78

(54) **Infektionsschutzvorrichtung für Endoexo-Implantate**
Infection prevention device for transcutaneous implants
Dispositif anti-infection pour implants transcutanés

(30) Priorität: 09.04.2003 DE 10316726
(43) Veröffentlichungstag der Anmeldung: 13.10.2004
(73) Patentinhaber: Affeld, Klaus, Prof. Dr. Ing., D-10629 Berlin (DE)
(72) Erfinder: Affeld, Klaus, Dr., 10629 Berlin (DE); Bagheri, Mohammed, 14050 Berlin (DE)
(74) Vertreter: Neumann, Günter

(56) Entgegenhaltungen:
- WO-A-00/76566
- DE-C- 19 852 848
- DE-U- 29 880 029
- US-A- 5 236 422
- GROSSE-SIESTRUP C ET AL: "DESIGN CRITERIA FOR PERCUTANEOUS DEVICES" JOURNAL OF BIOMEDICAL MATERIALS RESEARCH, WILEY, NEW YORK, NY, US, Bd. 18, Nr. 1, 1984, Seiten 357-382, XP000878714 ISSN: 0021-9304

## Beschreibung

Die Erfindung betrifft eine Infektionsschutzvorrichtung für Endoexo-Implantate.

Endoexo-Implantate sind Implante die auf der einen Seite im Körperinneren verankert sind und sich auf der anderen Seite außerhalb des Körpers befinden. Dies bedeutet, daß die Endoexo-Implantate die Haut durchdringen. An dieser Hautdurchtrittsstelle können Mikroben eintreten und sich entlang der Oberfläche des Endoexo-Implantats bewegen und ins Körperinneren eindringen. Hierbei besteht die Gefahr, daß Infektionen auftreten. Durch sorgfältige Pflege der Stelle der Hautdurchdringung kann man diese zwar in Grenzen beherrschen, jedoch müssen immer wieder Endoexo-Implantate wegen Infektionen explantiert werden.

Ein Beispiel für ein Endoexo-Implantat ist der Fixateur Extern, der für die Stabilisierung von Knochenbrüchen eingesetzt wird. Hierbei wird eine Metallschraube in den Knochen eingeschraubt. Das andere Ende der Schraube, das außerhalb des Körpers liegt, wird mit einem Bügel verschraubt. Meist werden je Bruch vier Schrauben eingesetzt. Der Bügel verbindet die beiden, durch den Bruch getrennten Enden des Knochens und stabilisiert so den Knochenbruch. Nach der Heilung werden die Schrauben wieder entfernt. Häufig behindert jedoch eine Infektion den Heilungsprozeß. Die Tatsache, daß vier Schrauben eingesetzt werden erhöht hierbei die Gefahr einer Infektion.

Ein anderes Beispiel ist ein Endoexo-Implantat für die postoperative Versorgung nach einer Amputation. Hier kann es vorkommen, daß die Amputation nur einen kurzen Stumpf gestattet, an dem eine herkömmliche Arm- oder Beinprothese nicht befestigt werden kann. Man kann jedoch mit einem Endoexo-Implantat das Problem lösen. Es wird mit seinem einen Ende im Knochen verankert und ragt mit dem anderen Ende aus dem Körper heraus. An diesem Ende kann man die Prothese befestigen. Auch hier besteht die Gefahr einer Infektion.

Ein weiteres Beispiel ist ein Endoexo-Implantat aus dem Bereich der Inneren Medizin. So erfordert die Peritonealdialyse eine dauerhaft in der Bauchwand implantierte Leitung. Mit ihrem einen Ende liegt sie im Bauchraum und mit dem anderen Ende ragt sie aus dem Körper heraus. Durch diese Leitung wird die Dialyseflüssigkeit von außen in den Körper eingeleitet und auch wieder herausgeleitet. Auch hier besteht die Gefahr einer Infektion.

Ein weiteres Beispiel ist ein Endoexo-Implantat aus dem Bereich der Kardiochirurgie. Bei den meisten künstlichen Herzunterstützungssystemen ist die Zuleitung von pneumatischer oder elektrischer Energie von außen erforderlich. Dies erfolgt ebenfalls durch eine Leitung, die an ihrem einen Ende mit dem implantierten Herzunterstützungssystem verbunden ist und mit ihrem anderen Ende mit der Energiequelle außerhalb des Körpers. Auch hier besteht die Gefahr einer Infektion.

In allen Fällen erfordert eine Infektion, die tief in den Körper eingedrungen ist, eine Operation zur Entfernung des Endoexo-Implantats.

Die Infektion an einem Endoexo-Implantat bildet sich zuerst an der Grenze, an der sich die drei Phasen Implantat, Körpergewebe und keimhaltige Außenwelt treffen. Dies ist der Bereich, an der das Endoexo-Implantat die Haut durchtritt. Die Keime können an dieser Hautdurchtrittsstelle zunächst durch häufige Reinigung und durch äußere Bakteriostatika beherrscht werden. Bei einem längeren Verweilen bilden viele Keime jedoch einen Biofilm auf der Oberfläche des Endoexo-Implantats. Dieser Biofilm ist eine Schicht von Keimen, die sich am Kanalmaterial anhaftet und sich gegen die Körperabwehr durch eine Schleimschicht schützt. Da der Körper diesen Biofilm nicht durchdringen kann, kann er die Infektion nicht wirksam bekämpfen. Der Biofilm hat weiterhin die Eigenschaft sich auszudehnen. Er wächst in Richtung der Nährstoffquelle, in diesem Fall in die Richtung des Körperinneren. Dadurch bildet sich eine Tasche, die schwierig zu reinigen ist und die leicht zu einer größeren Infektion führen kann.

In der Offenlegungsschrift DE 37 29 253 A1 wird zur Vermeidung des Infektionsrisikos eine Manschette am Endoexo-Implantat im Bereich des Hautdurchtritts angebracht. Diese Manschette ist in der Lage, Antibiotika abzugeben und so die Mikroben zu bekämpfen. Die Erfahrung zeigt jedoch, daß diese Vorrichtung nur kurze Zeit wirksam bleibt, weil sich eine Resistenz der Mikroben herausbildet.

Weiterhin sind Vorrichtungen bekannt geworden, bei denen Manschetten mit Silber oder mit Silberverbindungen beschichtet worden sind. Aber auch hier hat man die Ausbildung von Resistenzen beobachtet.

In der Patentschrift DE 197 28 489 A1 wird die Katheteroberfläche mit einem organischen Gewebe aus Kollagenfasern oder Kollagen-Polymerfasern beschichtet. Die Idee ist, daß das natürliche Kollagen sich günstig auf das Einwachsen und dauerhafte Gedeihen des natürlichen Körpergewebes auswirkt.Die Erfahrung zeigt jedoch, daß auch diese Maßnahme Infektionen nicht verhindern kann.

Weiterhin sind Infektionsschutzvorrichtungen bekannt, bei denen man durch eine leichtbewegliche Struktur an der Hautdurchtrittsstelle die Einwirkung von mechanischen Kräften auf die anhaftenden Zellen verringern möchte. Dies ist näher dargestellt in einer Arbeit des Anmelders (Große-Siestrup, Ch., Affeld, K.: Design criteria for artificial percutaneous devices, Journal of Biomedical Materials Research, Vol 18, 357-382 (1984)). Die Erfahrung zeigt jedoch, dass auch diese Maßnahme auf Dauer eine Infektion nicht verhindern kann.

In der Patentschrift DE 198 52 848 A1 wird die Katheteroberfläche mit einem Infektionsschutzmantel umgeben, der im Körper gebildet wird und gleichförmig oder in Schüben herausgeschoben wird. Es liegen jedoch noch keine Erfahrungen über die Wirksamkeit dieser Maßnahme vor. Weiterhin kann dieser im Körper sich bildende Infektionsschutzmantel nicht mit den modernen Methoden der Biomaterialtechnik wie beispielsweise der Plasmabehandlung in seiner Bioverträglichkeit verbessert werden. Diese Methoden erfordern, dass das Material in ein Vakuum gebracht wird oder aber hohen Temperaturen ausgesetzt wird.

Allen diesen Infektionsschutzvorrichtungen ist gemeinsam, dass sie auf Dauer eine Infektion und ein Eindringen von Keimen in den Körper nicht verhindern können. Sie sind mit Komplikationen verbunden und gefährden den Patienten.

Der Erfindung liegt die Aufgabe zugrunde, die oben erwähnten Nachteile der bekannten Lösungen zu vermeiden und eine Infektionsschutzvorrichtung bereitzustellen, die auf technisch bessere Weise ein Eindringen von Keimen in den Körper verhindert.

Erfindungsgemäß wird die Aufgabe durch eine Infektionsschutzvorrichtung für ein Endoexo-Implantat mit einer schlauchförmige Schutzmembran, die sich aus dem sterilen Bereich des Körperinneren heraus in den unsterilen Außenbereich bewegt und so an der Hautdurchtrittsstelle eine Wandbewegung von innen nach außen erzeugt, die das gegenläufige Eindringen von Mikroben verhindert, wobei die schlauchförmige Schutzmembran gefaltet oder aufgerollt ausgebildet ist, an ihrem körperinneren Ende fest und hermetisch dicht mit dem Endoexo-Implantat verbunden ist und sich im Körperinnern beim Herausbewegen mittels einer im Außenbereich angeordneten Zugvorrichtung nach der Art einer Rollmembran abrollt oder ausfaltet.

Dadurch wird erreicht, dass das körperfremde Material in der Form der schlauchförmigen Schutzmembran sich an der infektionsgefährdeten Hautdurchtrittsstelle aus dem Körper herausbewegt. In technisch vorteilhafter Weise wird so ein Vorgang nachgeahmt, der im Körper auf natürliche Weise erfolgt, so beim Herauswachsen von unbelebten natürlichen Bildungen, wie von Haaren und Fingernägeln. Der sich im Bereich der Hautdurchtrittsstelle bildende Biofilm wird auf diese Weise aus dem Körper herausbewegt und kommt in nährstoffarme und trockene Bereiche, in denen er abstirbt. Diese schlauchförmige Schutzmembran umgibt also das eigentliche funktionstragende Element - beispielsweise eine Knochenschraube - und trennt es vom umgebenden Gewebe.

Vorteihafte Ausgestaltungen der erfindungsgemäßen Infektionsschutzvorrichtung ergeben sich aus den Merkmalen der Ansprüche 2 bis 4.

Die schlauchförmige Schutzmembran wird im Körperinneren wie eine Rollmembran abgerollt, oder ist im Körperinneren gefaltet oder wird aus einem Reservoir im Körperinneren herausgezogen. Dabei ist die Länge der faltbaren Schutzmembran grundsätzlich begrenzt und damit auch der Zeitraum, währenddessen sie herausgezogen werden kann. Dies ist jedoch bei den meisten Anwendungen kein Nachteil, weil der Zeitraum durch andere, nämlich medizinische Gründe ohnehin begrenzt ist.

Dem Material der schlauchförmigen Schutzmembran können ferner Substanzen beigemischt werden, die biologisch wirksam sind und die das Endoexo-Implantat vor Infektionen schützen.

So können Antibiotika beigemischt werden, die dann im Körper durch Diffusion ins umgebende Gewebe eindringen und es sicher keimfrei halten. Der Gefahr der oben angeführten Resistenzbildung kann dadurch begegnet werden, dass ringweise wechselnde Antibiotika aufgebracht werden. Andere Substanzen können das Anhaften der Zellen an der schlauchförmigen Schutzmembran fördern. Das Anhaften der Zellen kann weiterhin durch eine vergrößerte Oberfläche gefördert werden.

Die schlauchförmige Schutzmembran, die in der oben beschriebenen Weise beständig oder in Abständen aus dem Körper herausbewegt wird, wird dann in Abständen - wiederum ähnlich den natürlichen Bildungen wie Haar und Fingernägel - mechanisch abgetrennt und so gekürzt oder auch aufgerollt.

Die mit der Erfindung erzielten Vorteile bestehen insbesondere auch darin, dass Infektionen im gesamten Bereich des Endoexo-Implantats vermieden werden können.

Im Folgenden soll die Erfindung an hand von Zeichnungen näher erläutert werden. Es zeigen:
- **Figur 1**: eine Ausbildung der Infektionsschutzvorrichtung für ein Endoexo-Implantat in einem Querschnitt eines Oberschenkels;
- **Figur 2**: ein Endoexo-Implantat in einem Längsschnitt;
- **Figur 3**: eine Verankerung in Draufsicht;
- **Figur 4**: ein weiteres Endoexo-Implantat in einem Längsschnitt.

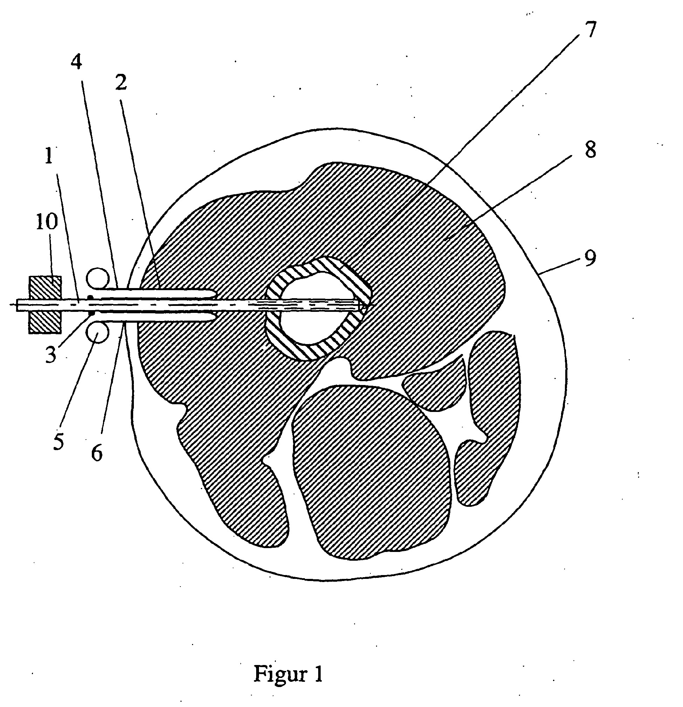

In **Fig. 1** ist als Endoexo-Implantat 1 eine Knochenschraube zur Stabilisierung eines außerhalb der Zeichnungsebene liegenden Knochenbruchs dargestellt. Die Schraube ist in den Knochen 7 eingeschraubt und durchdringt die Muskeln 8 und weiterhin die Haut 9 und reicht an der Hautdurchtrittsstelle 6 in den Außenbereich. Der ganze Bereich im Körperinnern kann bei sachgemäßer Implantation als steril betrachtet werden. Der Außenbereich muss dagegen als unsteril betrachtet werden. An der Schraube ist an der Stelle 3 die schlauchförmige Schutzmembran 2 hermetisch befestigt, so dass hier keine Mikroben hindurchdringen können. Durch die Zugvorrichtung 5 kann die schlauchförmige Schutzmembran 2 aus dem Körper herausbewegt werden und rollt sich so nach der Art einer Rollmembran ab. Gewöhnlich werden vier Knochenschrauben mit einem gemeinsamen Bügel 10 verbunden und stabilisieren auf diese Weise den Knochenbruch.

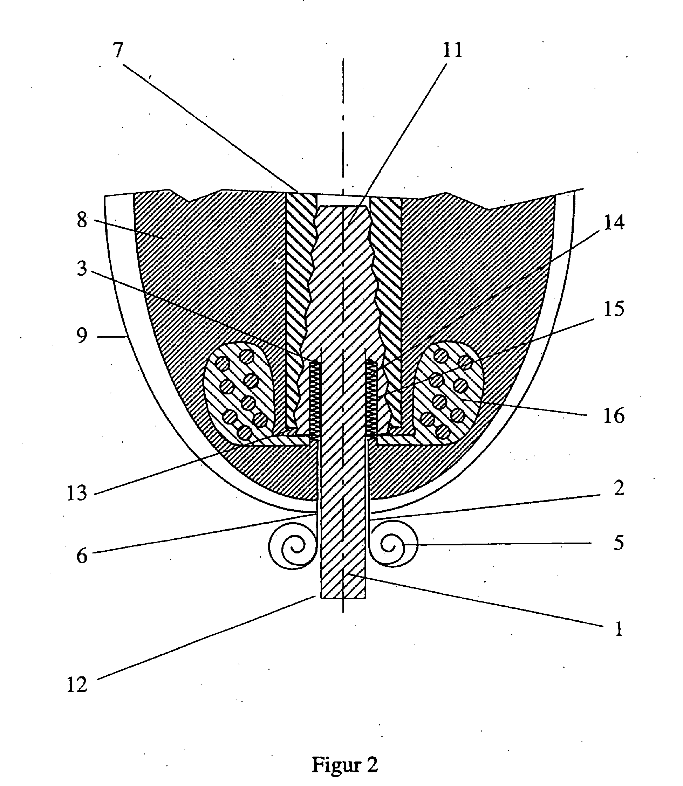

**Fig. 2** zeigt ein Endoexo-Implantat in einem Längsschnitt. Hier handelt es sich um ein Endoexo-Implantat zur Befestigung einer Beinprothese an einem Stumpf. Das Endoexo-Implantat 1 ist hier in seinen lasttragenden Elementen mehrteilig aufgebaut. An seinem proximalen Ende 11 ist es fest und formschlüssig mit dem Röhrenknochen 7 verbunden, mit seinem distalen Ende 12 ragt es in die Außenwelt. Der Röhrenknochen 7 ist an der Stelle 13 amputiert worden. Das distale Ende 12 des Endoexo-Implantats 1 ist mit einem Prothesenreiniger 11 verbunden, der die Haut durchdringt und an seinem Ende mit der Prothese verbunden werden kann. Das Endoexo-Implantat 1 ist an seinem distalen Ende 12 mit einer schlauchförmigen Schutzmembran 2 umgeben, die hier in zusammengefalteter Form 14 in der Ausnehmung 15 untergebracht ist und damit in das Körperinnere hereinreicht. An der Stelle 3 ist die schlauchförmige Schutzmembran 2 wiederum hermetisch dicht mit dem Endoexo-Implantat 1 verbunden. Die schlauchförmige Schutzmembran 2 wird von der Zugvorrichtung 5 mit definierter Geschwindigkeit aus der Ausnehmung 15 gezogen. An der Hautdurchtrittsstelle 6 durchtritt die schlauchförmige Schutzmembran 2 die Haut und verlässt somit den Körper. Durch die Restmuskeln im Stumpf können an der Hautdurchtrittsstelle 6 Kräfte wirken, die auf die schlauchförmige und von Zellen bewachsene Schutzmembran 2 wirken. Diese Kräfte können einen Spalt erzeugen, in den Mikroben eindringen können. Um diese Einwirkung von Kräften zu vermeiden, ist eine Verankerung 16 vorgesehen. Sie soll die von den Restmuskeln erzeugten Kräfte aufnehmen und in den Knochen ableiten. Die Verankerung 16 wird bei der Operation durch eine Spaltung des Gewebes eingesetzt und wird dann mit dem Endoexo-Implantat 1 mechanisch fest verbunden. So werden auftretende Kräfte in den Knochen abgeleitet und können nicht auf die Hautdurchtrittsstelle 6 wirken. Zur besseren Aufnahme der Kräfte ist die Verankerung 16 mit einer geeigneten Oberfläche versehen und besitzt durchwachsbare Durchbrüche.

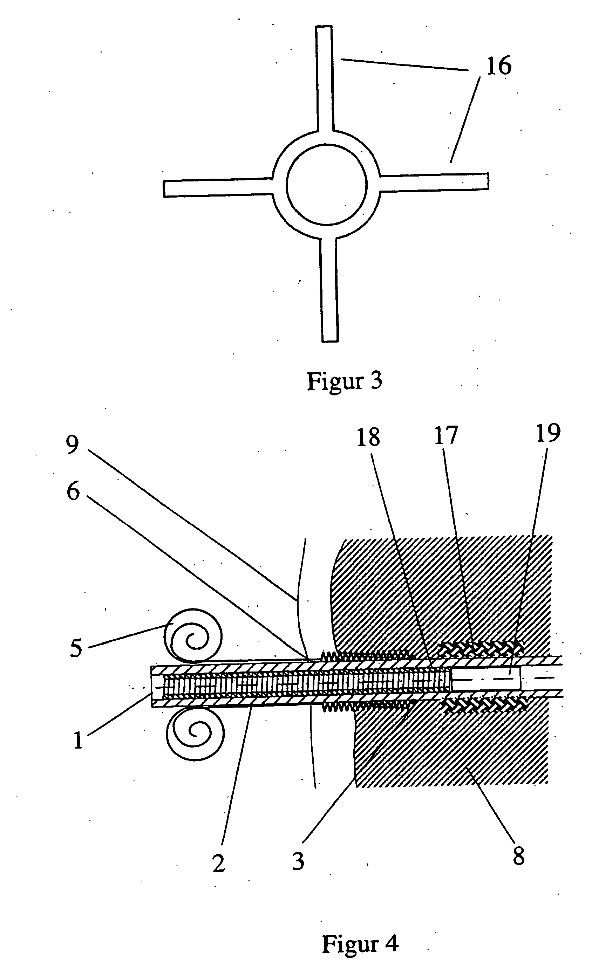

**Fig. 3** zeigt die Verankerung 16 in einer Aufsicht. Die Verankerung hat mehrere Flügel, an denen Gewebe anwachsen kann. Die Hautdurchtrittsstelle 6 wie in Fig. 2 dargestellt kann so von Kräften freigehalten werden.

**Fig. 4** zeigt ein weiteres Endoexo-Implantat 1 in einem Längsschnitt. Hier handelt es sich um ein Endoexo-Implantat aus dem Bereich der Inneren Medizin. Es handelt sich um eine dauerhaft in der Bauchwand implantierte Leitung. Mit ihrem einen Ende liegt sie im Bauchraum und mit dem anderen Ende ragt sie aus dem Körper heraus. Durch diese Leitung wird die Dialyseflüssigkeit von außen in den Körper eingeleitet und auch wieder herausgeleitet. Die schlauchförmige Schutzmembran 2 ist im Inneren des Körpers auf der Stelle 3 hermetisch dicht mit der Leitung 1 verbunden. Die schlauchförmige Schutzmembran 2 umgibt die Leitung 1 in gefalteter Form. Dies hat den Zweck, eine möglichst große Länge der schlauchförmigen Schutzmembran 2 im sterilen Bereich des Körpers zu halten, damit eine möglichst lange Funktionszeit erreicht werden kann. Durch den Zug der Zugvorrichtung 5 wird die Faltung aufgehoben und die schlauchförmige Schutzmembran 2 verlässt den Körper an der Hautdurchtrittsstelle 6 in gestreckter Form. Die Leitung 1 besitzt eine durchwachsbare Manschette 17, die sie im Gewebe oder Muskel 8 fixiert. Der Zug auf die schlauchförmige Schutzmembran 2 durch die Zugvorrichtung 5 wird durch ein flexibles aber drucksteifes Element 18 und über ein starres Rohr 19 durch die flexible Wand der Leitung 1 auf die durchwachsbare Manschette 17 übertragen.

## Patentansprüche

1. Infektionsschutzvorrichtung für ein Endoexo-Implantat (1) mit einer schlauchförmige Schutzmembran (2), die sich aus dem sterilen Bereich des Körperinneren heraus in den unsterilen Außenbereich bewegt und so an der Hautdurchtrittsstelle (6) eine Wandbewegung von innen nach außen erzeugt, die das gegenläufige Eindringen von Mikroben verhindert, **dadurch gekennzeichnet, dass** die schlauchförmige Schutzmembran (2) gefaltet oder aufgerollt ausgebildet ist, an ihrem einen Ende (3) fest und hermetisch dicht mit dem Endoexo-Implantat (1) verbunden ist und sich im Körperinnern beim Herausbewegen mittels einer im Außenbereich angeordneten Zugvorrichtung (5) nach der Art einer Rollmembran abrollt oder ausfaltet.

2. Vorrichtung nach Anspruch 1, **dadurch gekennzeichnet, dass** das Herausbewegen der schlauchförmigen Schutzmembran (2) aus dem Körperinneren automatisch und definiert erfolgt.

3. Vorrichtung nach einem der voranstehenden Ansprüche, **dadurch gekennzeichnet, dass** die schlauchförmige Schutzmembran (2) in ihrer Oberfläche und Materialeigenschaft so beschaffen ist, dass sie von körpereigenen Zellen bewachsbar ist.

4. Vorrichtung nach einem oder mehreren der voranstehenden Ansprüche, **dadurch gekennzeichnet, dass** das Material der schlauchförmigen Schutzmembran (2) biologisch wirksame Stoffe enthält.

## Claims

1. Infection protection device for an endoexo-implantation (1) with a tube-shaped protective membrane (2), which moves out of the sterile area of the internal body into the unsterile external area and so causes a wall movement at the point of penetration of the skin (6) from the inside outwards which hinders the opposite penetration of microbes **characterized by** the tube-shaped protective membrane (2) being folded or rolled up, attached permanently and hermetically with its end (3) to the endoexo-implantation (1) and is rolled out or unfolded in the inside of the body when moved out by means of a pulling device (5) on the outside in the form of a roll-membrane.

2. Device in accordance with claim 1, **characterized by** the moving out of the tube-shaped protective membrane (2) being performed automatically from the inside of the body.

3. Device in accordance with one of the above claims, **characterized by** the surface and material properties of the tube-shaped protective membrane (2) being such that it can grow into the body's own cells.

4. Device in accordance with one or more of the above claims, **characterized by** the material of the tube-shaped protective membrane (2) containing biologically active substances.

## Revendications

1. Dispositif anti-infection destiné à un implant transcutané (1) muni d'une membrane protectrice tubulaire (2) se déplaçant de la zone stérile située à l'intérieur du corps dans la zone extérieure non stérile, générant ainsi au point de pénétration dans la peau (6) un déplacement de la paroi de l'intérieur vers l'extérieur empêchant la pénétration des microbes dans le sens opposé, **caractérisé en ce que** la membrane protectrice tubulaire (2) présente une configuration pliée ou enroulée, est reliée à l'une de ses extrémités (3) fixement et hermétiquement à l'implant transcutané (1) et se déroule ou se déplie à l'intérieur du corps à la façon d'une membrane enroulée lors de son extraction à l'aide d'un dispositif de traction (5) disposé dans la zone extérieure.

2. Dispositif selon la revendication 1, **caractérisé en ce que** l'extraction de la membrane protectrice tubulaire (2) hors du corps humain s'effectue automatiquement et de façon définie.

3. Dispositif selon l'une quelconque des revendications précédentes, **caractérisé en ce que** la nature de la surface et de la matière composant la membrane protectrice tubulaire (2) permet à celle-ci d'être recouverte de cellules endogènes.

4. Dispositif selon l'une quelconque ou plusieurs revendications précédentes, **caractérisé en ce que** la matière composant la membrane protectrice tubulaire (2) comprend des substances à effet biologique.
